# FASCICULE DE BREVET EUROPEEN

(11) **EP 2 388 031 B1**
(45) Date de publication et mention de la délivrance du brevet: **06.09.2017**
(21) Numéro de dépôt: 11163805.2
(22) Date de dépôt: 27.04.2011
(51) Int. Cl.: A61M 5/14, A61M 5/168

(54) **Système de préparation et/ou d'administration de fluides de traitement médical**
Vorbereitungs- und/oder Verabreichungssystem von Flüssigkeiten zur medizinischen Behandlung
System for preparing and/or administering medical treatment fluids

(30) Priorité: 27.04.2010 FR 1053195
(43) Date de publication de la demande: 23.11.2011
(73) Titulaire: Doran International, 69780 Toussieu (FR)
(72) Inventeur: Buisson, Philippe, 69780 Toussieu (FR)
(74) Mandataire: Verriest, Philippe

(56) Documents cités:
- WO-A1-99/10028
- WO-A1-2009/106977
- DE-A1- 4 336 336

## Description

La présente invention concerne un système de préparation et/ou d'administration de fluides de traitement médical.

Une ligne de perfusion pour l'administration de fluides de traitement médical à un patient, tels que des produits de chimiothérapie, comporte de façon connue :
- un robinet comprenant une voie d'entrée de fluide comportant des moyens de connexion à une seringue, et une voie de sortie de fluide, et
- un prolongateur dont l'une des extrémités, dite proximale, est montée sur la voie de sortie de fluide et dont l'autre extrémité, dite distale, est équipée de moyens de connexion à un cathéter.

Le procédé d'utilisation d'une telle ligne de perfusion comprend les étapes suivantes consistant à :
- connecter une première seringue de rinçage contenant un produit de rinçage, tel que du sérum physiologique ou du glucose, à la voie d'entrée de fluide du robinet,
- purger le robinet et le prolongateur en injectant dans ces derniers une quantité suffisante du produit de rinçage contenu dans la seringue de rinçage,
- déconnecter la seringue de rinçage,
- connecter une seringue d'administration contenant un fluide de traitement médical sur la voie d'entrée de fluide du robinet,
- injecter le fluide de traitement médical jusqu'au cathéter à l'aide de la seringue d'administration, et
- déconnecter le prolongateur du cathéter.

L'utilisation d'une telle ligne de perfusion implique des étapes dangereuses pour le personnel hospitalier lorsque le fluide de traitement médical à administrer au patient est un produit de chimiothérapie, du fait notamment de la manipulation des récipients contenant ces produits par le personnel hospitalier, et des risques de projections de produit de chimiothérapie ou d'inspiration de vapeurs de ce produit lors de la déconnexion du prolongateur du cathéter.

De plus, lors du transport d'un produit de chimiothérapie entre ses zones de préparation et d'administration, le récipient contenant ce dernier peut être brisé ou contaminé, et le transporteur est susceptible d'entrer en contact avec des particules de produit de chimiothérapie déposées sur les parois extérieures du récipient.

En outre, du fait que le produit de chimiothérapie est préparé longtemps avant son administration à un patient, il peut être obligatoire pour le personnel hospitalier d'agiter le récipient contenant ce produit afin d'assurer un mélange homogène des différents produits constitutifs de ce dernier, ce qui augmente les risques de contamination du personnel hospitalier et les risques d'une mauvaise administration au patient.

Le document DE 43 36 336 divulgue un système d'administration de fluides de traitement médical comprenant un dispositif d'actionnement de pompe et de clapet B2 auquel sont montées une cartouche équipée d'une pompe d'infusion A2, une cartouche formant collecteur D, et une cartouche d'application de médicaments E.

Le dispositif d'actionnement de pompe et de soupape B2 comprend notamment une pluralité de réservoirs d'administration 101-105 montés à la face extérieure du dispositif d'actionnement de pompe et de clapet, des premiers et seconds moyens de commande agencés pour commander l'ouverture et la fermeture des différents clapets équipant respectivement la cartouche d'application de médicaments E et la cartouche formant collecteur D, et des troisièmes moyens de commande agencés pour commander la compression de la chambre de compression 47 et la vidange de celle-ci.

Le système d'administration décrit dans le document DE 43 36 336 présente sensiblement les mêmes inconvénients que la ligne de perfusion précédemment décrite, du fait notamment du montage des réservoirs d'administration à la face extérieure du dispositif d'actionnement de pompe et de clapet.

La présente invention vise à remédier à tout ou partie de ces inconvénients.

Le problème technique à la base de l'invention consiste donc à fournir un système de préparation et/ou d'administration de fluides de traitement médical qui soit de structure simple et économique, et dont l'utilisation permet de préserver le personnel hospitalier et un transporteur de tout contact avec les liquides de traitement destinés à être administrés à un patient.

A cet effet, l'invention concerne un système de préparation et/ou d'administration de fluides de traitement médical comprenant :
- au moins un module de transport dans lequel sont logés au moins un réservoir d'administration destiné à contenir un fluide de traitement médical à administrer à un patient, une ligne d'administration équipée d'une entrée et d'une sortie de fluide accessibles depuis l'extérieur du module de transport et à laquelle est relié chaque réservoir d'administration, et des moyens d'obturation associés à chaque réservoir d'administration, les moyens d'obturation associés à chaque réservoir d'administration étant mobiles entre une première position dans laquelle le réservoir d'administration correspondant est isolé fluidiquement de la ligne d'administration et une seconde position dans laquelle le réservoir d'administration correspondant est relié fluidiquement à la ligne d'administration, et
- au moins une unité de préparation et/ou d'administration sur laquelle est destiné à être positionné le module de transport, l'unité de préparation et/ou d'administration comportant des premiers moyens de commande agencés pour commander les déplacements des moyens d'obturation associés à chaque réservoir d'administration entre leurs première et seconde positions.

Le système selon l'invention se distingue de l'art antérieur en ce que, d'une part, il comporte au moins un réservoir d'administration logé dans le module de transport, et d'autre part, en ce que le module de transport est destiné à être positionné sur l'unité de préparation et/ou d'administration. Ainsi, du fait que les différents réservoirs d'administration sont stockés dans le module de transport, le personnel hospitalier et un éventuel transporteur ne peuvent pas entrer en contact avec les fluides de traitement médical à administrer à un patient, et donc être contaminés par ces derniers.

En outre, ces dispositions permettent de protéger les réservoirs durant leur transport entre leurs zones de préparation et d'administration, et également de préserver la propreté de ces derniers.

Il convient de noter qu'une unité peut être utilisée uniquement pour réaliser le remplissage des différents réservoirs contenus dans un module de transport ou uniquement pour réaliser l'administration de fluides de traitements médicaux à un patient. Dans ce cas, le système selon l'invention comporte avantageusement au moins une unité de préparation de fluides sur laquelle est destiné à être positionné un module de transport de manière à remplir chaque réservoir d'administration avec un fluide de traitement médical, et au moins une unité d'administration de fluides sur laquelle est destiné à être positionné le module de transport de manière à administrer à un patient les différents fluides de traitements médicaux contenus dans les réservoirs d'administration de ce dernier.

Il convient de noter qu'une unité peut être utilisée à la fois pour réaliser le remplissage des différents réservoirs contenus dans un module de transport et pour réaliser l'administration de fluides de traitements médicaux à un patient. L'unité est alors identifiée comme une unité de préparation et d'administration.

De préférence, les premiers moyens de commande sont agencés pour commander les déplacements des moyens d'obturation associés à chaque réservoir d'administration uniquement dans une position de fonctionnement du système de préparation et/ou d'administration dans laquelle le module de transport est positionné sur l'unité de préparation et/ou d'administration.

Selon un mode de réalisation, les premiers moyens de commande comportent des moyens de contrôle et des moyens d'actionnement pilotés par les moyens de contrôle et agencés pour coopérer mécaniquement avec les moyens d'obturation associés à chaque réservoir d'administration lorsque le module de transport est positionné sur l'unité de préparation et/ou d'administration et pour déplacer ces derniers entre leurs première et seconde positions.

Avantageusement, les moyens d'obturation associés à chaque réservoir d'administration comportent une vanne 3 voies.

Selon un autre mode de réalisation, les premiers moyens de commande sont agencés pour être couplés électriquement avec les moyens d'obturation associés à chaque réservoir d'administration lorsque le module de transport est positionné sur l'unité de préparation et/ou d'administration. Selon ce mode de réalisation, le module de transport pourrait comporter un contacteur électrique destiné à coopérer avec un contacteur électrique disposé sur l'unité de préparation et/ou d'administration lorsque le module de transport est positionné sur cette dernière.

De façon avantageuse, le module de transport est réalisé sous la forme d'un boîtier de transport.

Avantageusement, le module de transport comporte un couvercle mobile entre des positions d'ouverture et de fermeture du module de transport, et des moyens de verrouillage du couvercle mobiles entre une position de verrouillage du couvercle dans sa position de fermeture et une position de déverrouillage du couvercle. Ces dispositions permettent d'empêcher toute manipulation des réservoirs d'administration durant leur transport.

De façon avantageuse, l'unité de préparation et/ou d'administration comprend des seconds moyens de commande agencés pour commander les déplacements des moyens de verrouillage entre leurs positions de verrouillage et de déverrouillage.

De façon préférentielle, le système de préparation et/ou d'administration comporte des moyens de verrouillage du module de transport sur l'unité de préparation et/ou d'administration.

Préférentiellement, le module de transport contient en outre au moins un réservoir de dilution relié à la ligne d'administration en amont des réservoirs d'administration et destiné à contenir un fluide de dilution, tel que du sérum physiologique ou du glucose, et des moyens d'obturation associés à chaque réservoir de dilution, les moyens d'obturation associés à chaque réservoir de dilution étant mobiles entre une première position dans laquelle le réservoir de dilution correspondant est isolé fluidiquement de la ligne d'administration et une seconde position dans laquelle le réservoir de dilution correspondant est relié fluidiquement à la ligne d'administration, et en ce que les premiers moyens de commande sont agencés pour commander les déplacements des moyens d'obturation associés à chaque réservoir de dilution entre leurs première et seconde positions.

De préférence, la ligne d'administration comprend une chambre de mélange disposée en aval des réservoirs d'administration et en amont de la sortie de fluide.

Selon une caractéristique de l'invention, le module de transport comporte une ligne de dérivation dont l'une des extrémités est reliée à la chambre de mélange et dont l'autre extrémité est reliée à la ligne d'administration en amont des réservoirs d'administration et en aval de l'au moins un réservoir de dilution, la ligne de dérivation comportant des moyens d'obturation mobiles entre une première position dans laquelle les moyens d'obturation empêchent un écoulement de fluide à travers la ligne de dérivation et une seconde position dans laquelle les moyens d'obturation autorise un écoulement de fluide à travers la ligne de dérivation, et les premiers moyens de commande sont agencés pour commander les déplacements des moyens d'obturation de la ligne de dérivation entre leurs première et seconde positions. Ces dispositions permettent de réaliser une dilution du ou des fluides contenus dans la chambre de mélange avant leur administration au patient, ce qui est avantageux lorsque le mélange n'est pas stable dans le temps.

De préférence, la ligne d'administration comporte une pompe volumétrique disposée entre les réservoirs d'administration et la chambre de mélange, et une pompe volumétrique disposée entre l'au moins un réservoir de dilution et les réservoirs d'administration.

De façon avantageuse, le module de transport comprend un réservoir de récupération relié à la ligne d'administration en amont de la sortie de fluide, et de préférence en aval de la chambre de mélange, et des moyens d'obturation mobiles entre une première position dans laquelle le réservoir de récupération est isolé fluidiquement de la ligne d'administration et une seconde position dans laquelle le réservoir de récupération est relié fluidiquement à la ligne d'administration, et en ce que les premiers moyens de commande sont agencés pour commander les déplacements des moyens d'obturation associés au réservoir de récupération entre leurs première et seconde positions.

Avantageusement, le module de transport est équipé d'un module d'identification, de préférence une puce RFID, et l'unité de préparation et/ou d'administration comporte des moyens de dialogue sans contact destinés à détecter et à identifier le module d'identification lorsque le module de transport est positionné sur l'unité de préparation et/ou d'administration. Ces dispositions permettent d'assurer une traçabilité du module de transport.

De préférence, l'unité de préparation et/ou d'administration comporte une unité centrale de traitement de données et des moyens de transfert de données destinés à transmettre des données caractéristiques d'un module d'identification détecté et identifié à l'unité centrale de traitement de données. Les moyens de transfert peuvent par exemple être des moyens de transfert sans fil, tel que des moyens de transfert du type wifi.

L'unité de préparation et/ou d'administration comporte avantageusement des moyens d'horodatage des cycles de remplissage des réservoirs d'administration logés dans le module de transport, et des cycles d'administration au patient des fluides de traitements médicaux contenus dans lesdits réservoirs.

Selon un mode de réalisation, l'unité de préparation et/ou d'administration comporte un lecteur de code-barres et une imprimante code-barres destinée à imprimer une étiquette à positionner sur le module de transport, le code-barres de cette dernière enfermant des données relatives au patient et au contenu de chaque réservoir (par exemple la nature et la quantité du produit contenu dans chaque réservoir, la date et l'heure de préparation du produit). Le code-barres de l'étiquette à positionner sur le module de transport peut également enfermer des données relatives à l'opérateur ayant préparé le produit.

Selon un mode de réalisation, le module de transport comprend des moyens de pressurisation agencés pour maintenir une pression prédéterminée sur les parois extérieures des réservoirs d'administration logés dans le module de transport.

De préférence, l'entrée de fluide est équipée de moyens de connexion, tels que de type luer, agencés pour permettre la connexion d'un contenant, tel qu'une seringue. Avantageusement, la sortie de fluide est équipée de moyens de connexion, tels que de type luer, agencés pour permettre la connexion d'un prolongateur dont l'une des extrémités est équipée de moyens de connexion à un élément formant cathéter.

Préférentiellement, l'unité de préparation et/ou d'administration comporte au moins une balance, et les premiers moyens de commande sont conçus pour commander le déplacement des moyens d'obturation associés à chaque réservoir d'administration, et en option à chaque réservoir de dilution, entre leurs première et seconde positions en fonction de la valeur mesurée par ladite balance.

Selon un mode de réalisation, la ligne d'administration comporte une chambre de contrôle disposée en aval de la chambre de mélange, la chambre de contrôle étant équipée de moyens de contrôle agencés pour contrôler la qualité du fluide issu de la chambre de mélange.

Avantageusement, le module de transport peut comporter des moyens d'isolation thermique afin de protéger thermiquement les fluides contenus dans les différents réservoirs logés dans le module de transport.

De toute façon l'invention sera bien comprise à l'aide de la description qui suit en référence au dessin schématique annexé représentant, à titre d'exemple non limitatif, une forme d'exécution de ce système de préparation et/ou d'administration.
Figures 1 et 2 sont des vues schématiques de face et de côté d'un système de préparation et/ou d'administration de fluides selon l'invention.
Figure 3 est une vue partielle, à l'échelle agrandie, du système de préparation et/ou d'administration de la figure 1.
Figures 4 et 5 sont des vues schématiques du module de transport du système de la figure 1 dans deux positions de fonctionnement différentes.

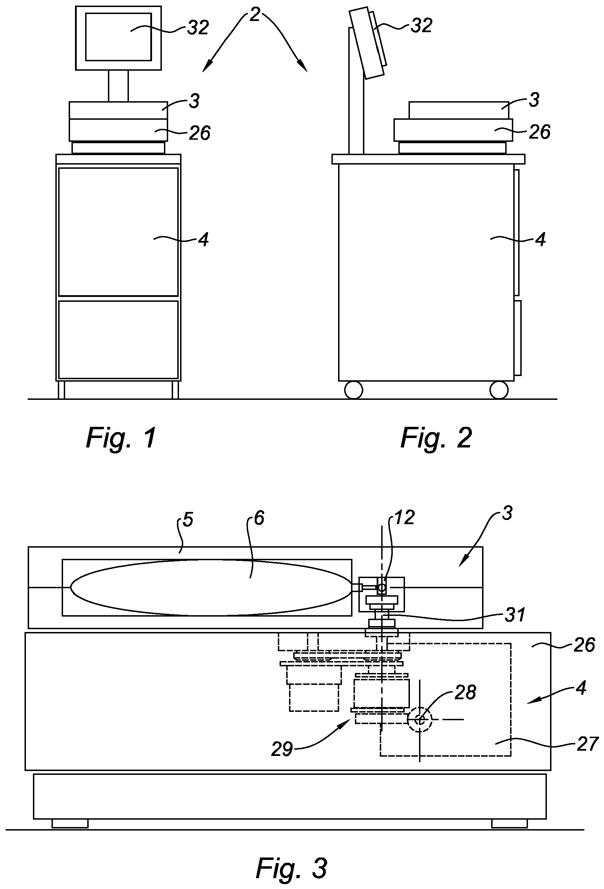

Les figures 1 et 2 représentent un système de préparation et d'administration de fluides 2 selon l'invention comprenant un module de transport 3, et une unité de préparation et d'administration de fluides 4 sur laquelle est destiné à être positionné le module de transport 3.

Comme montré plus particulièrement sur la figure 3, le module de transport 3 est réalisé sous la forme d'un boîtier de transport équipé d'un couvercle 5 mobile entre des positions d'ouverture et de fermeture du boîtier de transport. Le boîtier de transport peut par exemple être réalisé en matière plastique.

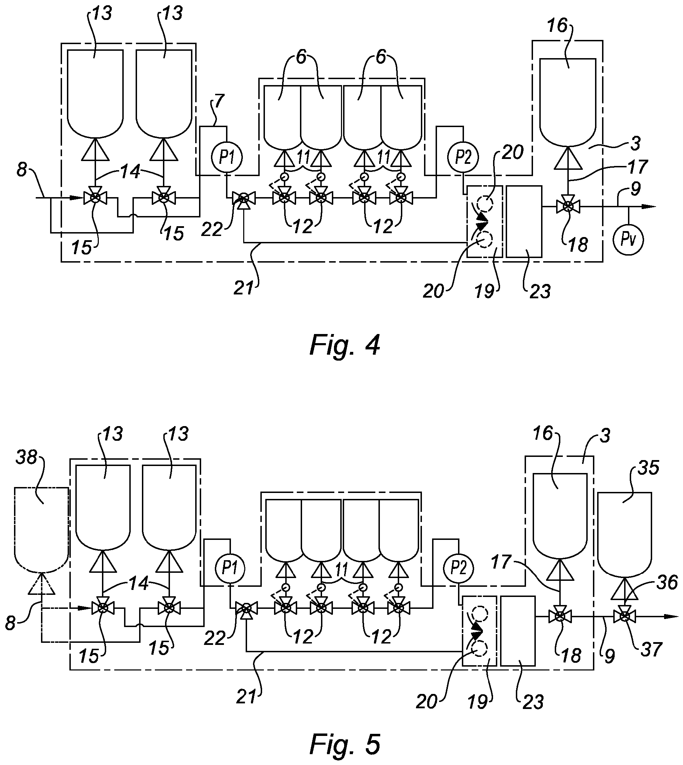

Comme montré sur les figures 4 et 5, le module de transport 3 contient une pluralité de réservoirs d'administration 6 destinés chacun à contenir un fluide de traitement médical à administrer à un patient, une ligne d'administration 7 équipée d'une entrée et d'une sortie de fluide 8, 9 accessibles depuis l'extérieur du module de transport 3, et une pluralité de lignes de raccordement 11 reliant chacune l'un des réservoirs d'administration 6 à la ligne d'administration 7.

Chaque ligne de raccordement 11 comporte une vanne 3 voies 12 mobile entre une première position dans laquelle le réservoir d'administration correspondant 6 est isolé fluidiquement de la ligne d'administration 7 et une seconde position dans laquelle le réservoir d'administration correspondant 6 est relié fluidiquement à la ligne d'administration 7.

De préférence, l'entrée de fluide 8 est équipée de moyens de connexion de type luer agencés pour permettre par exemple la connexion d'un contenant, tel qu'une seringue, et la sortie de fluide 9 est équipée de moyens de connexion de type luer agencés pour permettre par exemple la connexion d'un prolongateur équipé de moyens de connexion à un élément formant cathéter.

Le module de transport 3 contient également deux réservoirs de dilution 13 destiné à contenir respectivement du sérum physiologique et du glucose, et deux lignes de dilution 14 reliant chacune l'un des réservoirs de dilution 13 à la ligne d'administration 7 en amont des réservoirs d'administration 6. Chaque ligne de dilution 14 comporte une vanne 3 voies 15 mobile entre une première position dans laquelle le réservoir de dilution correspondant 13 est isolé fluidiquement de la ligne d'administration 7 et une seconde position dans laquelle le réservoir de dilution correspondant 13 est relié fluidiquement à la ligne d'administration 7.

Le module de transport 3 contient en outre un réservoir de récupération 16, et une ligne de récupération 17 reliant le réservoir de récupération 16 à la ligne d'administration 7 en amont de la sortie de fluide 9. La ligne de récupération 17 comporte une vanne 3 voies 18 mobile entre une première position dans laquelle le réservoir de récupération 16 est isolé fluidiquement de la ligne d'administration 7 et une seconde position dans laquelle le réservoir de récupération 16 est relié fluidiquement à la ligne d'administration 7

La ligne d'administration 7 comprend une chambre de mélange 19 disposée en aval des réservoirs d'administration 6 et en amont du réservoir de récupération 16, la chambre de mélange 19 comportant avantageusement des moyens de mélange 20 des différents fluides contenus dans cette dernière.

Le module de transport 3 comporte de plus une ligne de dérivation 21 dont l'une des extrémités est reliée à la chambre de mélange 19 et dont l'autre extrémité est reliée à la ligne d'administration 7 en amont des réservoirs d'administration 6 et en aval des réservoirs de dilution 13. La ligne de dérivation 21 comporte une vanne 3 voies 22 mobile entre une première position dans laquelle un écoulement de fluide à travers la ligne de dérivation 21 est empêché et une seconde position dans laquelle un écoulement de fluide à travers la ligne de dérivation 21 est autorisé.

La ligne d'administration 7 comporte en outre une pompe volumétrique P1 disposée entre les réservoirs de dilution 13 et les réservoirs d'administration 6, et une pompe volumétrique P2 disposée entre les réservoirs d'administration 6 et la chambre de mélange 19. Ces pompes volumétriques P1, P2 permettent de réguler le débit d'écoulement dans la ligne d'administration 7 des différents fluides contenus dans les réservoirs d'administration et de dilution.

La ligne d'administration 7 comporte également de façon avantageuse une chambre de contrôle 23 disposée entre la chambre de mélange 19 et le réservoir de récupération 16, la chambre de contrôle 23 étant équipée de moyens de contrôle (non représentés sur les figures) agencés pour contrôler la qualité du fluide issu de la chambre de mélange 19.

De préférence, les différents réservoirs 6, 13, 16 contenus dans le module de transport 3 sont souples, et avantageusement constitués par des poches de perfusion.

Le module de transport 3 comporte également des moyens de verrouillage (non représentés sur les figures) du couvercle 5 mobiles entre une position de verrouillage du couvercle dans sa position de fermeture et une position de déverrouillage du couvercle.

Selon un mode de réalisation de l'invention, le module de transport 3 pourrait comprendre, à la place des pompes volumétriques P1, P2 ou en complément de ces dernières, des moyens de pressurisation (non représentés sur les figures) agencés pour maintenir une pression prédéterminée sur les parois extérieures des réservoirs d'administration 6 logés dans le module de transport.

L'unité de préparation et d'administration 4 est réalisée sous la forme d'une servante mobile et comporte des premiers moyens de commande agencés pour commander indépendamment les déplacements des vannes 3 voies des lignes de récupération, de dérivation, de raccordement et de dilution entre leurs première et seconde positions. Les premiers moyens de commande sont aptes à commander les déplacements des différentes vannes 3 voies uniquement dans une position de fonctionnement du système de préparation et d'administration 2 dans laquelle le module de transport 3 est positionné sur l'unité de préparation et d'administration 4.

Les premiers moyens de commande comportent un boîtier de commande 26 contenant des moyens de contrôle (non représentés sur les figures), et des moyens d'actionnement pilotés par les moyens de contrôle et agencés d'une part pour coopérer mécaniquement avec les vannes 3 voies de chaque ligne de raccordement lorsque le module de transport 3 est positionné sur l'unité de préparation et d'administration 4, et d'autre part pour déplacer ces dernières entre leurs première et seconde positions. Les moyens de contrôle comportent avantageusement un microprocesseur. Les moyens d'actionnement comportent de préférence un moteur électrique 27 dont l'arbre de sortie est conçu pour entraîner en rotation une vis sans fin 28 couplée à un système d'embrayage 29, le système d'embrayage 29 étant conçu pour entraîner en rotation une pluralité d'organes d'actionnement 31 destinés chacun à coopérer avec l'une des vannes 3 voies du module de transport 3 de manière à faire pivoter cette dernière entre ces première et seconde positions.

Le microprocesseur et le moteur électrique 27 sont avantageusement alimentés en énergie électrique par le secteur et/ou par une batterie de secours logée par exemple dans le boîtier de commande 26 et destinée à rendre l'unité de préparation et d'administration 4 autonome et insensible à toute coupure de courant.

L'unité de préparation et d'administration 4 comporte également un lecteur de code-barres et une imprimante code-barres destinée à imprimer une étiquette à positionner sur le module de transport 3, le code-barres de cette dernière enfermant des données relatives notamment au patient et au contenu de chaque réservoir du module de transport 3.

L'unité de préparation et d'administration 4 comporte des moyens d'affichage et de saisie, tels qu'un écran tactile 32 , et une unité centrale de traitement de données logée dans la servante et comportant des moyens de stockage de données destinés à stocker les données saisies à l'aide de l'écran tactile 32 et du lecteur de code-barres.

L'unité de préparation et d'administration 4 comporte également des seconds moyens de commande (non représentés sur les figures) agencés pour commander les déplacements des moyens de verrouillage du couvercle 5 du module de transport 3 entre leurs positions de verrouillage et de déverrouillage lorsque ce dernier est positionné sur l'unité de préparation et d'administration 4.

L'unité de préparation et d'administration 4 comporte en outre des moyens de verrouillage du module de transport 3 sur cette dernière.

L'unité de préparation et d'administration 4 peut avantageusement comporter au moins une balance électronique. Les premiers moyens de commande sont alors conçus pour commander le déplacement des vannes 3 voies de chaque lignes de dilution et d'administration entre leurs première et seconde positions, en fonction de la valeur mesurée par ladite balance.

Selon un mode de réalisation de l'invention, le module de transport 3 est équipé d'un module d'identification, de préférence une puce d'identification RFID, et l'unité de préparation et d'administration 4 comporte des moyens de dialogue sans contact destinés à détecter et à identifier le module d'identification porté par un module de transport 3 lorsque ce dernier est positionné sur l'unité de préparation et d'administration 4, et des moyens de transfert de données destinés à transmettre des données caractéristiques d'un module d'identification détecté et identifié à l'unité centrale de traitement de données. Les données transférées par les moyens de transfert sont stockées dans les moyens de stockage de données de l'unité centrale.

Selon un mode de réalisation de l'invention, l'unité de préparation et d'administration 4 comprend des moyens d'avertissement sonore et/ou visuel indiquant à un opérateur tout dysfonctionnement de l'unité et/ou du module de transport.

Selon un mode de réalisation de l'invention, l'unité centrale comprend des moyens d'horodatage des données transférées à l'unité centrale. Il est par conséquent possible d'enregistrer précisément la date et l'heure de début et de fin de chaque cycle de préparation et d'administration, et la date et l'heure de chaque positionnement et retrait d'un module de transport sur ladite unité de préparation et d'administration 4.

Il va maintenant être décrit un procédé de préparation de fluides. Il convient de noter qu'en position initiale les différentes vannes 3 voies contenues dans un module de transport 3 sont de préférence dans leur première position.

Le procédé de préparation de fluides comprend les étapes suivantes consistant à :
a) prévoir une unité de préparation et d'administration 4,
b) identifier le patient à l'aide des moyens de saisie 3 ou du lecteur de code-barres de ladite unité 4,
c) positionner un module de transport 3 sur l'unité de préparation et d'administration 4,
d) verrouiller le module de transport 3 sur l'unité de préparation et d'administration 4,
e) verrouiller le couvercle 5 du module de transport 3,
f) relier une pompe à vide Pv à la sortie de fluide 9 du module de transport 3 et mettre à vide la ligne d'administration 7,
g) identifier un récipient, tel qu'une seringue, équipé d'un code-barres et contenant une quantité prédéterminée d'un premier liquide de traitement médical à l'aide du lecteur de code-barres, et raccorder ledit récipient à l'entrée de fluide 8 du module de transport 3,
h) commander le déplacement de la vanne 3 voies 12 de l'une des lignes de raccordement 11 dans sa seconde position, remplir le réservoir d'administration correspondant 6 à l'aide du premier liquide de traitement médical, commander le déplacement de ladite vanne 3 voies 12 dans sa première position, et déconnecter le récipient de l'entrée de fluide 8 du module de transport,
i) identifier un récipient, tel qu'une seringue, équipé d'un code-barres et contenant une quantité prédéterminée d'un fluide de rinçage à l'aide du lecteur de code-barres, raccorder ledit récipient à l'entrée de fluide 8 du module de transport 3, commander le déplacement de la vanne 3 voies 18 de la ligne de récupération 17 dans sa seconde position, rincer la ligne d'administration 7 à l'aide du fluide de rinçage, commander le déplacement de la vanne 3 voies 18 de la ligne de récupération dans sa première position, et déconnecter le récipient de l'entrée de fluide 8 du module de transport 3,
j) répéter les étapes g) à i) de manière à remplir les différents réservoirs d'administration 6 à l'aide de différents liquides de traitements médicaux,
k) raccorder un récipient, tel qu'une seringue, contenant une quantité prédéterminée de sérum physiologique à l'entrée de fluide 8 du module de transport,
l) commander le déplacement de la vanne 3 voies 15 de l'une des lignes de dilution 14 dans sa seconde position, remplir le réservoir de dilution correspondant 13 de sérum physiologique, commander le déplacement de ladite vanne 3 voies 15 dans sa première position, et déconnecter le récipient de l'entrée de fluide 8 du module de transport 3,
m) raccorder un récipient, tel qu'une seringue, contenant une quantité prédéterminée de glucose à l'entrée de fluide 8 du module de transport,
n) commander le déplacement de la vanne 3 voies 15 de l'autre ligne de dilution 14 dans sa seconde position, remplir le réservoir de dilution correspondant 13 de glucose, commander le déplacement de ladite vanne 3 voies dans sa première position, et déconnecter le récipient de l'entrée de fluide 8 du module de transport 3,
o) imprimer une étiquette à l'aide de l'imprimante code-barres, et positionner ladite étiquette sur le module de traitement 3,
p) identifier l'étiquette positionnée sur le module de transport 3 à l'aide du lecteur de code-barres,
q) déverrouiller la module de transport 3 de l'unité de préparation et d'administration 4.

Selon un mode de mise en oeuvre du procédé de préparation, ce dernier comprend une étape préalable à l'étape g) qui consiste à raccorder une pompe volumétrique à l'entrée de fluide 8 de manière à faciliter le remplissage des différents réservoirs logés dans le module de transport, et plus particulièrement les réservoirs de dilution 13.

Selon un mode de mise en oeuvre du procédé de préparation, ce dernier comprend une étape préalable à chaque étape d'identification d'un récipient qui consiste à peser ledit récipient à l'aide de la balance électronique, le déplacement de la vanne 3 voies de la ligne de dilution ou d'administration correspondante dans sa seconde position étant commandé uniquement si le poids du récipient est égal à une valeur prédéterminée.

Selon un mode de mise en oeuvre du procédé de préparation, l'étape de déconnection d'un récipient de l'entrée de fluide 8 du module de transport 3 peut être réalisée avant l'étape de commande du déplacement de la vanne 3 voies de la ligne correspondante dans sa première position.

Il va maintenant être décrit un procédé d'administration de fluides à un patient.

Le procédé d'administration de fluides comprend les étapes suivantes consistant à :
a) prévoir une unité de préparation et d'administration 4,
b) positionner un module de transport 3 dont les réservoirs ont été préalablement remplis sur l'unité de préparation et d'administration 4,
c) identifier l'étiquette positionnée sur le module de transport 3 à l'aide du lecteur de code-barres de ladite unité,
d) identifier le patient à l'aide des moyens de saisie ou du lecteur de code-barres de ladite unité,
e) verrouiller le module de transport 3 sur l'unité de préparation et d'administration 4,
f) relier un prolongateur à la sortie de fluide 9 du module de transport 3,
g) purger la ligne d'administration 7 et le prolongateur à l'aide d'un fluide de dilution contenu dans l'un des réservoirs de dilution 13 en commandant le déplacement de la vanne 3 voies correspondante 15 dans sa seconde position, puis commander le déplacement de ladite vanne 3 voies dans sa première position,
h) connecter le prolongateur à un élément formant cathéter relié à un patient,
i) commander le déplacement de la vanne 3 voies 12 de l'une des lignes de raccordement 11 dans sa seconde position, administrer au patient le fluide de traitement médical contenu dans le réservoir d'administration correspondant 6, et commander le déplacement de ladite vanne 3 voies dans sa première position,
j) commander le déplacement de la vanne 3 voies de l'une des lignes de dilution 14 dans sa seconde position, rincer la ligne d'administration 7 et le prolongateur à l'aide du fluide contenu dans le réservoir de dilution correspondant 13, et commander le déplacement de ladite vanne 3 voies 15 dans sa première position,
k) répéter les étapes i) et j) de manière administrer au patient les différents fluides de traitement médical contenus dans les différents réservoirs d'administration 6,
l) enregistrer la date et l'heure de fin du cycle d'administration à l'aide des moyens d'horodatage,
m) déverrouiller le couvercle 5 du module de transport 3 et déverrouiller la module de transport 3 de l'unité de préparation et d'administration 4,
n) déconnecter le prolongateur de la sortie de fluide 9.

Selon un mode de mise en oeuvre du procédé de préparation, ce dernier comprend une étape préalable à chaque étape d'administration d'un fluide de traitement médical au patient qui consiste à diluer ledit fluide de traitement médical dans la chambre de mélange 19 avec l'un des fluides de dilution contenus dans les réservoirs de dilution 13, puis à mélanger ces deux fluides dans la chambre de mélange 19 à l'aide des moyens de mélange 20.

Selon un mode de mise en oeuvre du procédé de préparation, ce dernier comprend une étape consistant à contrôler dans la chambre de contrôle 23 la qualité du fluide issu de la chambre de mélange 19 avant son administration au patient, et à autoriser cette administration uniquement si la qualité du fluide est satisfaisante.

Selon un autre mode de mise en oeuvre du procédé de préparation, ce dernier comprend une étape consistant à relier un réservoir de secours 35 contenant un fluide de rinçage à la sortie de fluide 9 du module de transport 3 par l'intermédiaire d'une ligne de secours 36 équipée d'une vanne 3 voies 37 déplaçable manuellement entre une première position dans laquelle le réservoir de secours 35 est isolé fluidiquement de la sortie de fluide 9 et une seconde position dans laquelle le réservoir de secours 35 est relié fluidiquement à la sortie de fluide 9. La présence d'un tel réservoir de secours 35 permet d'assurer un rinçage du prolongateur en cas de panne du module de transport 3 et/ou de l'unité de préparation et d'administration 4.

Selon encore un autre mode de mise en oeuvre du procédé de préparation, ce dernier comprend une étape consistant à relier un réservoir 38 contenant un fluide de dilution à l'entrée de fluide 8 du module de transport 3. La présence d'un tel réservoir permet de pallier un éventuel manque de fluide de dilution à l'intérieur du module de transport 3.

Comme il va de soi, l'invention ne se limite pas à la seule forme d'exécution de ce système de préparation et/ou d'administration de fluides, décrite ci-dessus à titre d'exemple, elle en embrasse au contraire toutes les variantes de réalisation, dans le cadre des revendications.

## Revendications

1. Système de préparation et/ou d'administration de fluides de traitement médical comprenant :
- au moins un module de transport (3) dans lequel sont logés au moins un réservoir d'administration (6) destiné à contenir un fluide de traitement médical à administrer à un patient, une ligne d'administration (7) équipée d'une entrée et d'une sortie de fluide (8, 9) accessibles depuis l'extérieur du module de transport et à laquelle est relié chaque réservoir d'administration, et des moyens d'obturation (12) associés à chaque réservoir d'administration, les moyens d'obturation (12) associés à chaque réservoir d'administration étant mobiles entre une première position dans laquelle le réservoir d'administration correspondant (6) est isolé fluidiquement de la ligne d'administration (7) et une seconde position dans laquelle le réservoir d'administration correspondant (6) est relié fluidiquement à la ligne d'administration (7), et
- au moins une unité de préparation et/ou d'administration (4) sur laquelle est destiné à être positionné le module de transport (3), l'unité de préparation et/ou d'administration comportant des premiers moyens de commande agencés pour commander les déplacements des moyens d'obturation (12) associés à chaque réservoir d'administration (6) entre leurs première et seconde positions.

2. Système selon la revendication 1, **caractérisé en ce que** les premiers moyens de commande sont agencés pour commander les déplacements des moyens d'obturation associés à chaque réservoir d'administration (6) uniquement dans une position de fonctionnement du système de préparation et/ou d'administration dans laquelle le module de transport (3) est positionné sur l'unité de préparation et/ou d'administration (4).

3. Système selon la revendication 1 ou 2, **caractérisé en ce que** les premiers moyens de commande comportent des moyens de contrôle et des moyens d'actionnement (27-31) pilotés par les moyens de contrôle et agencés pour coopérer mécaniquement avec les moyens d'obturation (12) associés à chaque réservoir d'administration (6) lorsque le module de transport (3) est positionné sur l'unité de préparation et/ou d'administration (4) et pour déplacer ces derniers entre leurs première et seconde positions.

4. Système selon l'une des revendications 1 à 3, **caractérisé en ce que** les moyens d'obturation associés à chaque réservoir d'administration (6) comportent une vanne 3 voies (12).

5. Système selon la revendication 1 ou 2, **caractérisé en ce que** les premiers moyens de commande sont agencés pour être couplés électriquement avec les moyens d'obturation (12) associés à chaque réservoir d'administration (6) lorsque le module de transport (3) est positionné sur l'unité de préparation et/ou d'administration (4).

6. Système selon l'une des revendications 1 à 5, **caractérisé en ce que** le module de transport (3) comporte un couvercle (5) mobile entre des positions d'ouverture et de fermeture du module de transport, et des moyens de verrouillage du couvercle mobiles entre une position de verrouillage du couvercle dans sa position de fermeture et une position de déverrouillage du couvercle.

7. Système selon la revendication 6, **caractérisé en ce que** l'unité de préparation et/ou d'administration comprend des seconds moyens de commande agencés pour commander les déplacements des moyens de verrouillage entre leurs positions de verrouillage et de déverrouillage.

8. Système selon la revendication l'une des revendications 1 à 7, **caractérisé en ce qu'**il comporte des moyens de verrouillage du module de transport (3) sur l'unité de préparation et/ou d'administration (4).

9. Système selon l'une des revendications 1 à 8, **caractérisé en ce que** le module de transport (3) contient en outre au moins un réservoir de dilution (13) relié à la ligne d'administration (7) en amont des réservoirs d'administration (6) et destiné à contenir un fluide de dilution, tel que du sérum physiologique ou du glucose, et des moyens d'obturation (15) associés à chaque réservoir de dilution (13), les moyens d'obturation (15) associés à chaque réservoir de dilution (13) étant mobiles entre une première position dans laquelle le réservoir de dilution correspondant est isolé fluidiquement de la ligne d'administration (7) et une seconde position dans laquelle le réservoir de dilution correspondant est relié fluidiquement à la ligne d'administration (7), et **en ce que** les premiers moyens de commande sont agencés pour commander les déplacements des moyens d'obturation (15) associés à chaque réservoir de dilution (13) entre leurs première et seconde positions.

10. Système selon l'une des revendications 1 à 9, **caractérisé en ce que** la ligne d'administration (7) comprend une chambre de mélange (19) disposée en aval des réservoirs d'administration (6) et en amont de la sortie de fluide (9) du module de transport.

11. Système selon les revendications 9 et 10, **caractérisé en ce que** le module de transport (3) comporte une ligne de dérivation (21) dont l'une des extrémités est reliée à la chambre de mélange (19) et dont l'autre extrémité est reliée à la ligne d'administration (7) en amont des réservoirs d'administration (6) et en aval de l'au moins un réservoir de dilution (13), la ligne de dérivation (21) comportant des moyens d'obturation (22) mobiles entre une première position dans laquelle les moyens d'obturation (22) empêchent un écoulement de fluide à travers la ligne de dérivation (21) et une seconde position dans laquelle les moyens d'obturation (22) autorise un écoulement de fluide à travers la ligne de dérivation (21), et **en ce que** les premiers moyens de commande sont agencés pour commander les déplacements des moyens d'obturation (22) de la ligne de dérivation (21) entre leurs première et seconde positions.

12. Système selon la revendication 10 ou 11, **caractérisé en ce que** la ligne d'administration (7) comporte une pompe volumétrique (P2) disposée entre les réservoirs d'administration (6) et la chambre de mélange (19).

13. Système selon l'une des revendications 9 à 12, **caractérisé en ce que** la ligne d'administration (7) comporte une pompe volumétrique (P1) disposée entre l'au moins un réservoir de dilution (13) et les réservoirs d'administration (6).

14. Système selon l'une des revendications 1 à 13, **caractérisé en ce que** le module de transport (3) comprend un réservoir de récupération (16) relié à la ligne d'administration (7) en amont de la sortie de fluide (9), et de préférence en aval de la chambre de mélange (19), et des moyens d'obturation (18) mobiles entre une première position dans laquelle le réservoir de récupération (16) est isolé fluidiquement de la ligne d'administration (7) et une seconde position dans laquelle le réservoir de récupération (16) est relié fluidiquement à la ligne d'administration (7), et **en ce que** les premiers moyens de commande sont agencés pour commander les déplacements des moyens d'obturation (18) associés au réservoir de récupération entre leurs première et seconde positions.

15. Système selon l'une des revendications 1 à 14, **caractérisé en ce que** le module de transport (3) est équipé d'un module d'identification, de préférence une puce RFID, et **en ce que** l'unité de préparation et/ou d'administration (4) comporte des moyens de dialogue sans contact destinés à détecter et à identifier le module d'identification lorsque le module de transport (3) est positionné sur l'unité de préparation et/ou d'administration.

## Patentansprüche

1. System zur Herstellung und/oder zur Verabreichung von Fluiden zur medizinischen Behandlung, umfassend:
- mindestens ein Transportmodul (3), in dem mindestens ein Verabreichungsbehälter (6) gelagert ist, der ausgelegt ist, um ein Fluid zur medizinischen Behandlung zur Verabreichung an einen Patienten zu enthalten, eine Verabreichungslinie (7), die mit einem Eintritt und einem Austritt von Fluid (8, 9) ausgestattet ist, die von der Außenseite des Transportmoduls zugänglich sind, und mit der jeder Verabreichungsbehälter verbunden ist, und Verschlussmittel (12), die mit jedem Verabreichungsbehälter assoziiert sind, wobei die Verschlussmittel (12), die mit jedem Verabreichungsbehälter assoziiert sind, zwischen einer ersten Position, in der der entsprechende Verabreichungsbehälter (6) fluidisch von der Verabreichungslinie (7) isoliert ist, und einer zweiten Position, in der der entsprechende Verabreichungsbehälter (6) fluidisch mit der Verabreichungslinie (7) verbunden ist, beweglich sind, und
- mindestens eine Einheit zur Herstellung und/oder Verabreichung (4), auf der das Transportmodul (3) positioniert werden soll, wobei die Einheit zur Herstellung und/oder Verabreichung erste Steuermittel aufweist, die angeordnet sind, um die Verschiebungen der Verschlussmittel (12), die mit jedem Verabreichungsbehälter (6) assoziiert sind, zwischen ihren ersten und zweiten Positionen zu steuern.

2. System nach Anspruch 1, **dadurch gekennzeichnet, dass** die ersten Steuermittel angeordnet sind, um die Verschiebungen der Verschlussmittel, die mit jedem Verabreichungsbehälter (6) assoziiert sind, nur in einer Funktionsposition des Systems zur Herstellung und/oder Verabreichung zu steuern, in der das Transportmodul (3) auf der Einheit zur Herstellung und/oder Verabreichung (4) positioniert ist.

3. System nach Anspruch 1 oder 2, **dadurch gekennzeichnet, dass** die ersten Steuermittel Kontrollmittel und Betätigungsmittel (27 - 31) aufweisen, die von den Kontrollmitteln geleitet werden und angeordnet sind, um mit den Verschlussmitteln (12) mechanisch zusammenzuarbeiten, die mit jedem Verabreichungsbehälter (6) assoziiert sind, wenn das Transportmodul (3) auf der Einheit zur Herstellung und/oder Verabreichung (4) positioniert ist, und um diese Letzteren zwischen ihrer ersten und zweiten Position zu verschieben.

4. System nach einem der Ansprüche 1 bis 3, **dadurch gekennzeichnet, dass** die Verschlussmittel, die mit jedem Verabreichungsbehälter (6) assoziiert sind, einen 3-Weg-Schieber (12) aufweisen.

5. System nach Anspruch 1 oder 2, **dadurch gekennzeichnet, dass** die ersten Steuermittel angeordnet sind, um elektrisch mit den Verschlussmitteln (12) gekoppelt zu ein, die mit jedem Verabreichungsbehälter (6) assoziiert sind, wenn das Transportmodul (3) auf der Einheit zur Herstellung und/oder Verabreichung (4) positioniert ist.

6. System nach einem der Ansprüche 1 bis 5, **dadurch gekennzeichnet, dass** das Transportmodul (3) einen Deckel (5), der zwischen Öffnungs- und Verschlusspositionen des Transportmoduls beweglich ist, und Verriegelungsmittel des Deckels aufweist, die zwischen einer Verriegelungsposition des Deckels in seiner Verschlussposition und einer Entriegelungsposition des Deckels beweglich sind.

7. System nach Anspruch 6, **dadurch gekennzeichnet, dass** die Einheit zur Herstellung und/oder Verabreichung zweite Steuermittel umfasst, die angeordnet sind, um die Verschiebungen der Verriegelungsmittel zwischen ihren Verriegelungs- und Entriegelungspositionen zu steuern.

8. System nach einem der Ansprüche 1 bis 7, **dadurch gekennzeichnet, dass** es Verriegelungsmittel des Transportmoduls (3) auf der Einheit zur Herstellung und/oder Verabreichung (4) aufweist.

9. System nach einem der Ansprüche 1 bis 8, **dadurch gekennzeichnet, dass** das Transportmodul (3) außerdem mindestens einen Verdünnungsbehälter (13) umfasst, der mit der Verabreichungslinie (7) vorgelagert von den Verabreichungsbehältern (6) verbunden und ausgelegt ist, um ein Verdünnungsfluid zu enthalten, wie z. B. ein physiologisches Serum oder Glukose, und Verschlussmittel (15), die mit jedem Verdünnungsbehälter (13) assoziiert sind, wobei die Verschlussmittel (15), die mit jedem Verdünnungsbehälter (13) assoziiert sind, zwischen einer ersten Position, in der der entsprechende Verdünnungsbehälter fluidisch von der Verabreichungslinie (7) isoliert ist, und einer zweiten Position, in der der entsprechende Verdünnungsbehälter fluidisch mit der Verabreichungslinie (7) verbunden ist, beweglich sind, und dadurch, dass die ersten Steuermittel angeordnet sind, um die Verschiebungen der Verschlussmittel (15) die mit jedem Verdünnungsbehälter (13) assoziiert sind, zwischen ihren ersten und zweiten Positionen zu steuern.

10. System nach einem der Ansprüche 1 bis 9, **dadurch gekennzeichnet, dass** die Verabreichungslinie (7) eine Mischkammer (19) umfasst, die sich nachgelagert von den Verabreichungsbehältern (6) und vorgelagert vom Fluidaustritt (9) des Transportmoduls befindet.

11. System nach Anspruch 9 und 10, **dadurch gekennzeichnet, dass** das Transportmodul (3) eine Ableitungslinie (21) aufweist, wobei eines der Enden mit der Mischkammer (19) verbunden ist, und wobei das andere Ende mit der Verabreichungslinie (7) vorgelagert von den Verabreichungsbehältern (6) und nachgelagert von mindestens einem Verdünnungsbehälter (13) verbunden ist, wobei die Abweichungslinie (21) Verschlussmittel (22) aufweist, die zwischen einer ersten Position, in der die Verschlussmittel (22) ein Ablaufen von Fluid über die Abweichungslinie (21) verhindern, und einer zweiten Position, in der die Verschlussmittel (22) ein Ablaufen von Fluid über die Abweichungslinie (21) erlauben, beweglich sind, und dadurch dass die ersten Steuermittel angeordnet sind, um die Verschiebungen der Verschlussmittel (22) der Ableitungslinie (21) zwischen ihrer ersten und zweiten Positionen zu steuern.

12. System nach Anspruch 10 oder 11, **dadurch gekennzeichnet, dass** die Verabreichungslinie (7) eine Verdrängerpumpe (P2) aufweist, die sich zwischen den Verabreichungsbehältern (6) und der Mischkammer (19) befindet.

13. System nach einem der Ansprüche 9 bis 12, **dadurch gekennzeichnet, dass** die Verabreichungslinie (7) eine Verdrängerpumpe (P1) aufweist, die sich zwischen dem mindestens einen Verdünnungsbehälter (13) und den Verabreichungsbehältern (6) befindet.

14. System nach einem der Ansprüche 1 bis 13, **dadurch gekennzeichnet, dass** das Transportmodul (3) einen Wiedergewinnungsbehälter (16) umfasst, der mit der Verabreichungslinie (7) vorgelagert vom Fluidaustritt (9) und vorzugsweise nachgelagert von der Mischkammer (19) verbunden ist, und Verschlussmittel (18), die zwischen einer ersten Position, in der der Wiedergewinnungsbehälter (16) fluidisch von der Verabreichungslinie (7) isoliert ist, und einer zweiten Position, in der der Wiedergewinnungsbehälter (16) fluidisch mit der Verabreichungslinie (7) verbunden ist, beweglich sind, und dadurch, dass die ersten Steuermittel angeordnet sind, um die Verschiebungen der Verschlussmittel (18), die mit dem Wiedergewinnungsbehälter assoziiert sind, zwischen ihren ersten und zweiten Positionen zu steuern.

15. System nach einem der Ansprüche 1 bis 14, **dadurch gekennzeichnet, dass** das Transportmodul (3) mit einem Identifizierungsmodul ausgestattet ist, vorzugsweise einem RFID-Chip, und dadurch, dass die Einheit zur Herstellung und/oder Verabreichung (4) Mittel zum kontaktlosen Dialog aufweist, die ausgelegt sind, um das Identifizierungsmodul nachzuweisen und zu identifizieren, wenn das Transportmodul (3) auf der Einheit zur Herstellung und/oder Verabreichung positioniert ist.

## Claims

1. A system for preparing and/or administering medical treatment fluids comprising:
- at least one transport module (3) in which are housed at least one administration reservoir (6) intended to contain a medical treatment fluid to be administered to a patient, an administration line (7) equipped with a fluid inlet and outlet (8, 9) accessible from the outside of the transport module and to which is connected each administration reservoir, and obturation means (12) associated with each administration reservoir, the obturation means (12) associated with each administration reservoir being moveable between a first position in which the corresponding administration reservoir (6) is fluidically isolated from the administration line (7) and a second position in which the corresponding administration reservoir (6) is fluidically connected to the administration line (7), and
- at least one preparation and/or administration unit (4) on which the transport module (3) in intended to be positioned, the preparation and/or administration unit including first control means laid out for controlling the displacements of the obturation means (12) associated with each administration reservoir (6) between their first and second positions.

2. The system according to claim 1, **characterized in that** the first control means are laid out so as to control the displacements of the obturation means associated with each administration reservoir (6) only in an operating position of the preparation and/or administration system in which the transport module (3) is positioned on the preparation and/or administration unit (4).

3. The system according to claim 1 or 2, **characterized in that** the first control means include monitoring means and actuation means (27-31) driven by the monitoring means and laid out so as to mechanically cooperate with the obturation means (12) associated with each administration reservoir (6) when the transport module (3) is positioned on the preparation and/or administration unit (4) and for displacing the latter between their first and second positions.

4. The system according to any one of claims 1 to 3, **characterized in that** the obturation means associated with each administration reservoir (6) include a 3-way valve (12).

5. The system according to claim 1 or 2, **characterized in that** the first control means are laid out so as to be electrically coupled with the obturation means (12) associated with each administration reservoir (6) when the transport module (3) is positioned on the preparation and/or administration unit (4).

6. The system according to any one of claims 1 to 5, **characterized in that** the transport module (3) includes a lid (5) moveable between opening and closing positions of the transport module, and means for locking the lid which are moveable between a position for locking the lid in its closing position and a position for unlocking the lid.

7. The system according to claim 6, **characterized in that** the preparation and/or administration unit comprises second control means laid out for controlling the displacements of the locking means between their locking and unlocking positions.

8. The system according to any one of claims 1 to 7, **characterized in that** it includes means for locking the transport module (3) on the preparation and/or administration unit (4).

9. The system according to any one of claims 1 to 8, **characterized in that** the transport module (3) further contains at least one dilution reservoir (13) connected to the administration line (7) upstream from the administration reservoirs (6) and intended to contain a dilution fluid, such as normal saline or glucose, and obturation means (15) associated with each dilution reservoir (13), the obturation means (15) associated with each dilution reservoir (13) being moveable between a first position in which the corresponding dilution reservoir is fluidically isolated from the administration line (7) and a second position in which the corresponding dilution reservoir is fluidically connected to the administration line (7), and **in that** the first control means are laid out so as to control the displacements of the obturation means (15) associated with each dilution reservoir (13) between their first and second positions.

10. The system according to any one of claims 1 to 9, **characterized in that** the administration line (7) comprises a mixing chamber (19) positioned downstream from the administration reservoirs (6) and upstream from the fluid outlet (9) of the transport module.

11. The system according to claims 9 and 10, **characterized in that** the transport module (3) includes a bypass line (21), one of the ends of which is connected to the mixing chamber (19) and the other end of which is connected to the administration line (7) upstream from the administration reservoirs (6) and downstream from said at least one dilution reservoir (13), the bypass line (21) including obturation means (22) which are moveable between a first position in which the obturation means (22) prevent fluid flow through the bypass line (21) and a second position in which the obturation means (22) allow fluid flow through the bypass line (21), and **in that** the first control means are laid out so as to control the displacements of the obturation means (22) of the bypass line (21) between their first and second positions.

12. The system according to claim 10 or 11, **characterized in that** the administration line (7) includes a volumetric pump (P2) positioned between the administration reservoirs (6) and the mixing chamber (19).

13. The system according to any one of claims 9 to 12, **characterized in that** the administration line (7) includes a volumetric pump (P1) positioned between said at least one dilution reservoir (13) and the administration reservoirs (6).

14. The system according to any one of claims 1 to 13, **characterized in that** the transport module (3) comprises a recovery reservoir (16) connected to the administration line (7) upstream from the fluid outlet (9) and preferably downstream from the mixing chamber (19), and obturation means (18) which are moveable between a first position in which the recovery reservoir (16) is fluidically isolated from the administration line (7) and a second position in which the recovery reservoir (16) is fluidically connected to the administration line (7), and **in that** the first control means are laid out for controlling the displacements of the obturation means (18) associated with the recovery reservoir between their first and second positions.

15. The system according to any one of claims 1 to 14, **characterized in that** the transport module (3) is equipped with an identification module, preferably a RFID chip, and **in that** the preparation and/or administration unit (4) includes contactless interactive means intended to detect and identify the identification module when the transport module (3) is positioned on the preparation and/or administration unit.
